# FASCICULE DE BREVET EUROPEEN

(11) **EP 0 565 684 B1**
(45) Date de publication et mention de la délivrance du brevet: **17.04.1996**
(21) Numéro de dépôt: 92922686.8
(22) Date de dépôt: 31.10.1992
(51) Int. Cl.: A61F 9/06, G02C 7/10, G02F 1/1335

(54) **DISPOSITIF ELECTRO-OPTIQUE A TRANSPARENCE VARIABLE**
ELEKTRO-OPTISCHE VORRICHTUNG MIT EINSTELLBARER DURCHLAESSIGKEIT
ELECTRO-OPTICAL DEVICE HAVING VARIABLE LIGHT TRANSMISSION

(30) Priorité: 05.11.1991 CH 3218/91; 17.12.1991 FR 9115768
(43) Date de publication de la demande: 20.10.1993
(73) Titulaire: ASULAB S.A., CH-2501 Bienne (CH)
(72) Inventeur: GRUPP, Joachim, CH-2003 Neuchâtel (CH)
(74) Mandataire: Caron, Gérard
(86) Numéro de dépôt international: EP9202503
(87) Numéro de publication internationale: WO9308774

(56) Documents cités:
- EP-A- 0 091 514
- EP-A- 0 157 744
- WO-A-87/06018
- FR-A- 2 293 188
- FR-A- 2 416 519
- GB-A- 2 169 417
- PATENT ABSTRACTS OF JAPAN vol. 011, no. 337 (P-633)5 Novembre 1987 & JP-A-62 121 421 (SHARP)

## Description

L'invention concerne un dispositif électro-optique à transparence variable et plus particulièrement un tel dispositif appliqué à des dispositifs de protection des yeux tels que des lunettes comprenant des verres formés par des cellules électro-optiques qui présentent des caractéristiques d'absorption de la lumière particulièrement élevées.

Le domaine d'application de l'invention comprend aussi bien les lunettes classiques de soleil ou médicales que des dispositifs de protection des yeux tels que des masques de soudage ou analogues.

La plupart des lunettes à transparence variable connues actuellement et utilisées notamment comme lunettes de soleil, sont de deux types.

Le premier type comprend les lunettes munies de verres dits verres photochromiques. Ces derniers sont chargés d'une substance photochromique telle que des sels d'argent et ont la propriété de passer, automatiquement et de façon réversible, d'un état quasi transparent en l'absence de lumière solaire à un état absorbant sous l'effet de cette lumière.

Toutefois, de par leur nature, ces lunettes ne permettent généralement d'absorber qu'une quantité de lumière maximum de l'ordre de 70 à 80% de la lumière ambiante et ceci seulement après un temps de réaction d'une dizaine de minutes.

De plus, le temps de régénération après que les verres ont cessé d'être irradiés est d'environ de 10 à 20 minutes et la transmission de ces verres dans l'état transparent n'est que d'environ 80% ces verres conservant de façon permanente un aspect légérement coloré.

Par ailleurs, ces lunettes sont pratiquement inefficaces dès qu'elles sont utilisées derrière une surface vitrée car ces verres photochromiques sont essentiellement sensibles aux ultraviolets. Ces verres sont aussi très sensibles à la température et seront plus absorbants à basse température qu'à haute température pour une même quantité de lumière ambiante.

Enfin, il est difficile, pour un même degré d'absorption, de réaliser de telles lunettes comportant des verres d'épaisseur inférieure' à quelques mm sans risquer d'atteindre la limite de solubilité des substances photochromiques et donc sans risquer d'entraîner la précipitation de ces dernières.

Tout ceci en fait des lunettes qui réagissent passivement et qui présentent un confort d'utilisation relatif dès que l'on souhaite obtenir des lunettes de soleil réagissant immédiatement et efficacement dans un grand nombres de situations, et notamment derrière une surface vitrée telle qu'un pare-brise de véhicule automobile.

Le second type comprend des lunettes dont les verres sont formés par des cellules électro-optiques telles que des cellules à cristal liquide, telles que les lunettes décrites dans le document PCT/IT87/00024 (WO-A-87/06018).

Il s'agit dans ce document de cellules à cristal liquide du type dichroïque à contraste positif associées à un circuit de commande automatique ou manuel qui permet de faire passer les cellules d'un état transparent à un état absorbant. Dans l'état transparent ces cellules transmettent environ 80% de la lumière ambiante tandis que dans l'état absorbant elles en transmettent environ 40%. Bien que ces lunettes présentent un avantage majeur par rapport aux lunettes photochromiques car elles sont actives et ont un temps de réaction quasi nul après que le circuit de commande a été activé, ce dernier pouvant être notamment activé par des moyens de détection de la lumière ambiante ou par un simple interrupteur de commutation, elles n'en présentent pas moins certains inconvénients.

En effet, ces lunettes présentent, d'une part, un coefficient d'absorption assez faible de sorte qu'en présence de lumière d'une forte intensité l'utilisateur est mal protégé et peut être incommodé. D'autre part, les lames de ces cellules doivent subir un traitement antiultraviolets afin d'empêcher une dégradation prématurée du cristal liquide, qui outre le fait d'être coûteux donne un aspect jaunâtre et peu esthétique aux lames des cellules dans leur état transparent.

Bien entendu, ce qui vient d'être dit à propos des lunettes est au moins en partie vrai pour d'autres dispositifs de protection des yeux, comme par exemple le filtre pour un masque de soudage qui est décrit dans le document FR-A-2 293 188 et qui comprend entre deux lames de verre protectrices un filtre UV, un filtre IR et derrière ces filtres un système d'absorption de la lumière visible constitué par une cellule à cristal liquide et deux polariseurs placés de part et d'autre de celle-ci.

D'autre part, on connaît également, grâce notamment au document GB-A-2 169 417, des lunettes médicales avec des verres qui sont constitués chacun d'une seule lentille à cristal liquide ou de deux lentilles juxtaposées et dont la distance focale peut être commandée par l'amplitude ou la fréquence de la tension appliquée entre les électrodes de la ou des cellules qui forment la ou les lentilles.

Pour être en plus à transparence variable, certaines de ces lunettes ont des verres qui comprennent une substance photochromique qui est dispersée dans l'une au moins des lames de la ou des cellules à cristal liquide ou qui recouvre cette lame. D'autres ont des verres qui sont munis d'une cellule à cristal liquide supplémentaire chargée d'absorber plus ou moins la lumière en fonction de son intensité.

Ces lunettes correspondent donc à l'un ou l'autre type de lunettes, notamment de lunettes de soleil, dont il a été question précédemment.

L'invention a pour but principal de remédier aux inconvénients de l'art antérieur susmentionné en fournissant un dispositif électro-optique à transparence variable qui combine les caractéristiques avantageuses de l'utilisation de verres photochromiques et de cellules électro-optiques.

A cet effet, l'invention a pour objet un dispositif électro-optique à transparence variable comprenant :
- au moins un verre formé par une cellule électro-optique comprenant une première lame et une seconde lame munies chacune d'une électrode de commande, et un cadre de scellement interposé entre les deux lames pour former un volume étanche dans lequel est emprisonné un matériau électro-optique,
- des moyens générateurs de tension reliés auxdites électrodes pour appliquer une tension variable sur ledit matériau afin de faire varier la transmission de la cellule, automatiquement ou manuellement, en fonction de la lumière ambiante, ce dispositif étant caractérisé en ce que la cellule comprend au moins un élément chargé d'une substance photochromique présentant une transmission qui varie de façon réversible en fonction de l'intensité de la lumière ambiante.

Grâce à ces caractéristiques, on réalise un dispositif électro-optique à transparence variable présentant deux niveaux d'absorption successifs avec deux vitesses de réaction différentes qui permettent une réaction immédiate et efficace à toute source lumineuse et qui, en outre, présentent un coefficient d'absorption élevé de la lumière ambiante.

On notera aussi que, de manière avantageuse, l'élément chargé d'une substance photochromique forme un écran contre les rayonnement ultraviolets de sorte que le matériau électro-optique est protégé contre ces derniers.

De plus, le fait de répartir l'absorption de la lumière ambiante entre le matériau électro-optique et un élément chargé d'une substance photochromique permet à ce dernier d'être chargé soit d'une quantité de ladite substance inférieure à celle normalement nécessaire soit de réaliser des verres de très faible épaisseur ce qui diminue avantageusement le poids de l'ensemble du dispositif.

On remarquera que notamment dans l'application d'un tel dispositif à un dispositif de protection des yeux tel que des lunettes, l'effet d'absorption des cellules électro-optiques et l'effet photochromique sont complémentaires dans de nombreuses situations courantes. Ainsi, par exemple, dans un véhicule automobile dans lequel l'absorption des verres photochromiques est quasiment nulle tandis que celle des cellules électro-optiques est normalement efficace. De même, à l'extérieur, lorsque l'utilisateur passe d'une zone ensoleillée à une zone partiellement dans l'ombre dans laquelle il souhaite être protégé, les cellules électro-optiques peuvent être aisément désactivées l'utilisateur ne bénéficiant alors que de l'effet d'absorption des verres photochromiques.

Selon un mode réalisation préféré, l'élément chargé d'une substance photochromique est formé par une des lames de la cellule électro-optique.

L'invention a également pour objet un tel dispositif disposé dans une structure de support pour former avec ce dernier un dispositif de protection des yeux.

D'autres avantages et caractéristiques de l'invention apparaîtront à la lecture de la description détaillée qui suit, de modes de réalisation de l'invention, donnée à titre d'exemple non limitatif, en liaison avec les dessins annexés dans lesquels :
- la figure 1 est une vue en perspective partiellement en arraché d'un dispositif électro-optique selon l'invention appliqué à une paire de lunettes ; et
- la figure 2 est une coupe schématique d'un dispositif électro-optique selon l'invention associé à un circuit de commande automatique de sa transparence équipant les lunettes de la figure 1.

La description du dispositif électro-optique selon l'invention qui suit va être faite dans le cadre d'une application de celui-ci à des lunettes à transparence variable mais il est bien entendu qu'un tel dispositif peut trouver d'autres applications intéressantes, par exemple, dans des masques de soudage ou analogues ou encore pour réaliser des vitres pour fenêtres.

En outre, bien que le matériau électro-optique utilisé dans la cellule du dispositif selon l'invention décrit ci-après soit un mélange de cristaux liquides, il va de soi que d'autres types de matériaux électro-optiques tels que notamment des matériaux électrochromiques peuvent être également utilisés.

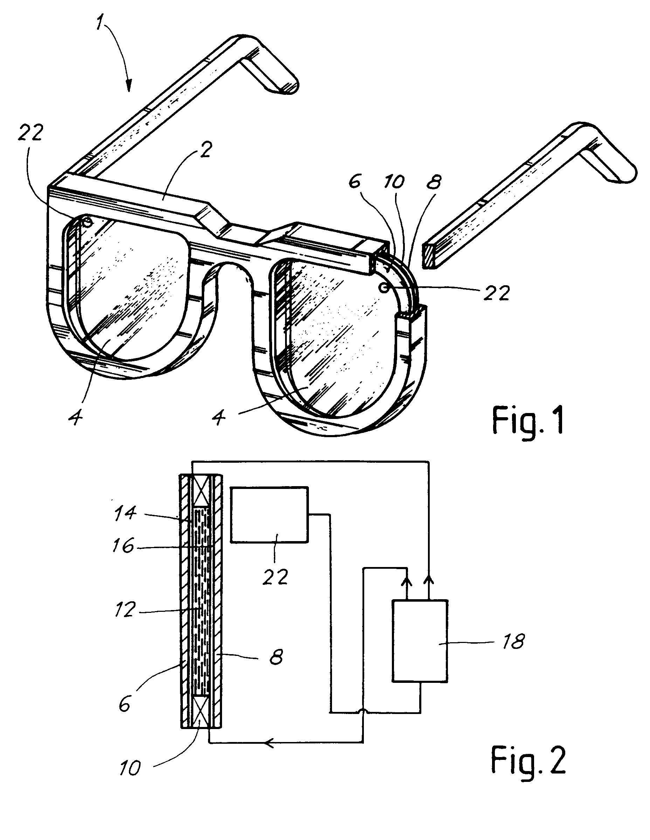

En se référant à la figure 1, on voit une paire de lunettes à cristal liquide à transparence variable selon l'invention et désignée par la référence générale 1. Ces lunettes sont généralement destinées à protéger la vue de leur utilisateur contre une lumière ambiante de forte intensité. Plus précisément ces lunettes comprennent une monture 2 dans laquelle sont enchassés de façon classique des verres 4 dont les caractéristiques d'absorption varient réversiblement en fonction de l'intensité de la lumière ambiante.

Ces verres 4 sont formés par des cellules à cristal liquide qui, comme cela ressort plus particulièrement de la figure 2, comprennent chacune une lame avant 6 et une lame arrière 8 transparentes réunies entre elles par un cadre de scellement 10. Ces deux lames 6, 8 et le cadre 10 définissent un volume étanche dans lequel est emprisonné un mélange 12 de cristaux liquides et de colorants dichroïques dopé d'un agent chiral.

Dans le mode de réalisation décrit, les lames 6 et 8 sont convexes et portent chacune sur leur face interne une électrode transparente 14, 16 recouverte d'une couche d'alignement (non représentée) qui s'étend sur la totalité de chaque lame. Les électrodes 14, 16 sont formées par exemple d'un mélange d'oxyde d'indium et d'étain, et la couche d'alignement est formée de dioxyde de silicium sur lequel est fixé un agent surfactant du type octadécytrialkoxysilane.

Il est à noter que les dessins ne représentent pas l'épaisseur exacte de l'ensemble ainsi formé, cette épaisseur étant fortement exagérée pour plus de clarté. Pour fixer les idées, la distance entre les deux électrodes est de l'ordre de 5 à 9 µm.

On peut voir également à la figure 2 que les cellules 4 sont associées à un circuit de commande électrique 18 alimenté par exemple par une pile ou des cellules solaires intégrées dans la monture 2 (non représentées). Ce circuit est relié à des moyens de détection de la lumière ambiante 22 formés par un ou plusieurs capteurs photosensibles qui délivrent un signal de commande électrique représentatif de l'intensité de la lumière qu'ils reçoivent. Ce circuit comprend en outre deux sorties reliées respectivement aux électrodes 14 et 16 afin de commander le degré d'absorption des cellules.

En d'autres termes, le signal élaboré par le circuit de commande 18 est appliqué aux électrodes des cellules pour faire varier le champ électrique appliqué au mélange 12 qu'elles contiennent. Cette variation a pour effet une modification de la transmission de la cellule, ce qui revient en définitive à une variation de son degré d'absorption de la lumière.

Dans le mode de réalisation représenté, les cellules électro-optiques 4 et le capteur 22 sont disposés l'un par rapport à l'autre de sorte qu'une lumière incidente ou que la lumière ambiante atteigne lesdites cellules en premier. Une telle disposition du ou des capteurs permet notamment, comme cela sera expliqué ci-après, d'obtenir une transmission constante dans une gamme de niveaux de lumière. Il va de soi que dans un autre mode de réalisation les moyens de détection peuvent être disposés afin d'être directement irradiés par les rayons lumineux que le dispositif selon l'invention est destiné à absorber.

Selon l'invention, chaque cellule comprend au moins un élément chargé d'une substance photochromique, ledit élément étant disposé sur la cellule de sorte que la lumière ambiante atteigne cet élément en premier lieu quand les lunettes sont portées par un utilisateur

Dans l'exemple décrit , ledit élément est formé par la lame 6 de la cellule 4. Cette lame est chargée d'une substance photochromique telle qu'un sel d'argent et peut être indifféremment une lame de verre ou en un matériau synthétique équivalent au verre pour ses propriétés optiques. Une lame en matériau synthétique réalisée en polyvinyle pyrolydine dopé avec des microcristaux de Ag-Cl est tout à fait appropriée. Cette lame de matériau synthétique peut encore être réalisée au moyen de films de polyester laminés sur une lame synthétique, par exemple une lame vendue sous la référence CR 39 par la société ESSILOR, à l'aide d'un adhésif dopé avec des spirooxazines photochromiques.

De préférence la concentration en substance photochromique n'excède pas 3% en poids et l'épaisseur dudit élément 6 est de l'ordre de 1 mm.. Selon une variante de l'invention, non représentée, ledit élément peut être formé par une lame supplémentaire directement rapportée sur la lame 6 des cellules, par exemple par collage.

Selon une autre variante (non représentée) de l'invention , les verres 4 formés par une cellule électro-optique peuvent être associés à une seconde cellule électro-optique dans une même structure de support afin de renforcer l'effet d'absorption du dispositif pour un dispositif de protection des yeux tel qu'un masque de soudage. Dans ce cas, la seconde cellule est placée derrière les verres 4 par rapport à la lumière amoiante et cette cellule peut être une cellule électro-optique à transparence variable classique.

A titre d'exemple illustratif et pour mieux démontrer les résultats avantageux de la combinaison des effets photochromique et électro-optique selon l'invention, la déposante a effectué des mesures comparatives de la transmission (en % de la lumière reçue) d'une cellule électro-optique seule, dans un état commuté et non commuté, d'un verre photochromique seul et du dispositif électro-optique selon l'invention dans un état commuté et non commuté, en présence et en l'absence de lumière comprenant des radiations UV.

Les mesures en présence de lumière ont été faites en présence d'une source lumineuse de 40mW/cm². La cellule électro-optique utilisée est une cellule à cristal liquide comprenant un mélange dichroïque vendu par la société MERCK sous la référence ZLI 4282, ce mélange étant dopé avec 0,85% en poids d'un agent chiral vendu par la société MERCK sous la référence S811.

Les substrats de cette cellule sont en verre. Les verres photochromiques utilisés sont des verres photochromiques standards que l'on peut par exemple obtenir auprès de la société Desag (Schott). On notera également que les résultats des mesures de la transmission du dispositif selon l'invention ont été obtenus avec un dispositif dans lequel l'élément chargé d'une substance photochromique est rapporté sur la cellule électro-optique par collage.

Les résultats obtenus sont consignés dans le tableau suivant :

| | TRANSMISSION % | |
|---|---|---|
| | sans lumière | avec lumière |
| Verres photochromiques | 75 à 85% | 20 à 30% |

| Cellule électro-optique | | |
|---|---|---|
| - non commutée | 80% | |
| - commutée | 40% | |

| Dispositif électro-optique selon l'invention | | |
|---|---|---|
| - non commuté | 70% | 19% |
| - commuté | 27% | 9% |

Il ressort de ce tableau que le dispositif selon l'invention présente un degré d'absorption qui peut varier dans une large gamme en fonction de l'intensité de la lumière ambiante.

En d'autres termes le dispositif selon l'invention peut devenir très absorbant dans le cas d'une importante luminosité et ne transmettre que 9% de la lumière ambiante, contre 20 et 40% avec un verre photochromique seul respectivement une cellule électro-optique à transparence variable (dans sont état commuté) seule.

De même, le dispositif selon l'invention peut tout aussi bien devenir très transmissif dans le cas d'une interruption totale de la luminosité et transmettre 70% de la lumière ambiante (dans l'état commuté de la cellule électro-optique). Un tel niveau de transmission est sensiblement égal au niveau de transmission des verres photochromiques et des cellules électro-optiques utilisés seuls.

Dans le cas où le dispositif électro-optique selon l'invention est intercalé entre les moyens de détection 22, et la source lumineuse la transparence du dispositif peut s'autoréguler de manière à être sensiblement constante pendant des périodes de transition correspondant notamment au passage d'un milieu sombre ou peu lumineux vers un milieu clair ou lumineux.

En effet, lors d'une telle transition, les moyens de détection recoivent dans un premier temps la quasitotalité de la lumière de sorte qu'ils commandent immédiatement l'obscurcissement de la cellule électro-optique alors que la substance photochromique n'a pas encore réagi. L'utilisateur a alors une protection immédiate.

Dans un second temps, c'est -à-dire quelque minutes après, l'élément photochromique, par exemple le substrat photochromique de la cellule, commence à réagir progressivement et absorbe à son tour une partie de la lumière ambiante. L'illumination des moyens de détection situés derrière la cellule est alors diminuée si bien que ces derniers engendrent un signal de commande qui augmente la transmission de la cellule au fur et à mesure que l'élément photochromique s'obscurcit.

Ainsi, on arrive à maintenir un degré de transmission constant du dispositif selon l'invention aussi bien lors du passage d'un milieu sombre vers un milieu lumineux que lors de faibles variations de luminosité du milieu.

Dans le cas contraire, c'est-à-dire dans le cas du passage d'un milieu lumineux vers un milieu plus sombre, la cellule se désactive immédiatement de sorte que le dispositif est rendu rapidement moins absorbant, puis l'élément photochromique perd progressivement sa capacité d'absorption jusqu'à atteindre un état complètement clair ou suffisamment clair pour que le dispositif puisse à nouveau s'autoréguler comme cela a été décrit précédemment.

## Revendications

1. Dispositif électro-optique à transparence variable comprenant :
- au moins un verre formé par une cellule électro-optique (4) comprenant une première lame (6) et une seconde lame (8) munies chacune d'une électrode (14, 16) de commande et un cadre de scellement (10) interposé entre les deux lames pour former un volume étanche dans lequel est emprisonné un matériau électro-optique (12),
- des moyens générateurs de tension (18) reliés auxdites électrodes pour appliquer une tension variable sur ledit matériau afin de faire varier la transmission de la cellule (4), automatiquement ou manuellement, en fonction de la lumière ambiante,
caractérisé en ce que la cellule (4) comprend au moins un élément (6) chargé d'une substance photochromique présentant une absorption qui varie de façon réversible en fonction de l'intensité de la lumière ambiante.

2. Dispositif selon la revendication 1, caractérisé en ce que ledit élément est formé par une des lames (6) de ladite cellule (4).

3. Dispositif selon la revendication 1, caractérisé en ce qu'il comprend deux éléments formés respectivement par la première (6) et la seconde (8) lame de ladite cellule (4).

4. Dispositif selon la revendication, 1 caractérisé en ce que ledit élément est formé par une lame supplémentaire directement rapportée sur une lame de ladite cellule.

5. Dispositif selon l'une des revendications précédentes, caractérisé en ce que ledit matériau électro-optique (12) est un mélange de cristaux liquides dichroïque à contraste positif.

6. Dispositif selon la revendication 5, caractérisé en ce que ledit mélange de cristaux liquides (12) est dopé avec un agent chiral.

7. Dispositif selon l'une des revendications 1 à 4, caractérisé en ce que ledit matériau électro-optique est un matériau électrochromique.

8. Dispositif selon l'une quelconque des revendications précédentes, caractérisé en ce que lesdites lames (6, 8) sont réalisées en un matériau synthétique.

9. Dispositif selon l'une quelconque des revendications précédentes, caractérisé en ce qu'il comprend en outre des moyens de détection (22) susceptibles de délivrer un signal de commande représentatif de l'intensité de la lumière atteignant ces moyens de détection (22), lesdits moyens générateurs (18) de tension agissant sur la transparence de la cellule (4) en réponse à ce signal de commande

10. Dispositif selon la revendication 9, caractérisé en ce que la cellule électro-optique (4) et lesdits moyens de détection (22) sont disposés l'un par rapport à l'autre de sorte qu'une lumière ambiante atteigne ladite cellule (4) en premier.

11. Dispositif de protection des yeux comprenant une structure de support (2) dans laquelle est monté un dispositif électro-optique à transparence variable (4), caractérisé en ce que ledit dispositif électro-optique (4) est conforme à l'une quelconque des revendications 1 à 10.

12. Dispositif de protection des yeux selon la revendication 11, caractérisé en ce qu'il comprend en outre une seconde cellule électro-optique montée dans ladite structure de support.

13. Dispositif de protection des yeux selon la revendication 11 ou 12, caractérisé en ce les moyens générateurs de tension sont formés par des cellules solaires.

## Claims

1. Variable transparency electro-optical device comprising:
- at least one lens formed by an electro-optical cell (4) comprising a first plate (6) and a second plate (8) each provided with a control electrode (14, 16) and a sealing frame (10) interposed between the two plates to form a sealed volume enclosing an electro-optical material (12),
- voltage generating means (18) connected to said electrodes to apply a variable voltage to said material in order to automatically or manually vary the transmission of the cell (4) as a function of the ambient light,
characterized in that the cell (4) comprises at least one element (6) charged with a photochromic substance having an absorption that varies in reversible manner as a function of the intensity of the light impinging on said cell.

2. Device according to Claim 1, characterized in that said element is formed by one of the plates (6) of said cell (4).

3. Device according to Claim 1, characterized in that it comprises two elements formed by the first (6) and second (8) plate respectively of said cell (4).

4. Device according to Claim 1, characterized in that said element is formed by an additional layer directly applied to a plate of said cell.

5. Device according to any one of the preceding claims characterized in that said electro-optical material (12) is a mixture of negative contrast dichroic liquid crystals.

6. Device according to Claim 5, characterized in that said mixture of liquid crystals (12) is doped with a chiral agent.

7. Device according to one of Claims 1 to 4, characterized in that said electro-optical material is an electrochromic material.

8. Device according to any one of the preceding claims, characterized in that said plates (6, 8) are made of a synthetic material.

9. Device according to any one of the preceding claims, characterized in that it also comprises detection means (22) capable of delivering a control signal dependant on the intensity of the light impinging on these detection means (22), said voltage generating means (18) acting on the transparency of the cell (4) in response to this control signal.

10. Device according to Claim 9, characterized in that the electro-optical cell (4) and said detection means (22) are disposed in relation to one another in such a manner that ambient light reaches said cell (4) first.

11. Device for protecting the eyes comprising a support structure (2) in which is mounted a variable transparency electro-optical device (4), characterized in that said electro-optical device (4) is according to any one of claims 1 to 10.

12. Device for protecting the eyes according to Claim 11, characterized in that it also comprises a second electro-optical cell mounted in said support structure.

13. Device for protecting the eyes according to Claims 11 or 12, characterized in that the voltage generating means are composed of solar cells.

## Patentansprüche

1. Elektro-optische Vorrichtung mit veränderlicher Transparenz, umfassend:
- wenigstens ein von einer elektro-optischen Zelle (4) gebildetes Glas, umfassend eine erste Scheibe (6) und eine zweite Scheibe (8), welche jeweils mit einer Steuerelektrode (14, 16) versehen sind, und ein Versiegelungsrahmen (10), der zwischen den beiden Scheiben angeordnet ist zur Bildung eines dichten Volumens, in welchem ein elektro-optisches Material (12) eingeschlossen ist,
- Mittel zur Spannungserzeugung (18), welche mit den Elektroden verbunden sind zur Anlage einer veränderlichen Spannung an das Material, damit die Transmission der Zelle (4), automatisch oder manuell, in Abhängigkeit von dem Umgebungslicht verändert wird,
dadurch gekennzeichnet, daß die Zelle (4) wenigstens ein mit einer photochromischen Substanz geladenes Element (6) umfaßt, das eine sich in Abhängigkeit von der Intensität des Umgebungslichtes auf reversible Weise verändernde Absorption aufweist.

2. Vorrichtung nach Anspruch 1, dadurch gekennzeichnet, daß das Element von einer der Scheiben (6) der Zelle (4) gebildet ist.

3. Vorrichtung nach Anspruch 1, dadurch gekennzeichnet, daß sie zwei Elemente umfaßt, die jeweils von der ersten (6) und der zweiten (8) Scheibe der Zelle (4) gebildet sind.

4. Vorrichtung nach Anspruch 1, dadurch gekennzeichnet, daß das Element von einer zusätzlichen Scheibe gebildet ist, die unmittelbar auf einer der Scheiben der Zelle angebracht ist.

5. Vorrichtung nach einem der vorhergehenden Ansprüche, dadurch gekennzeichnet, daß das elektro-optische Material (12) eine Mischung dichroider Flüssigkristalle mit positivem Kontrast ist.

6. Vorrichtung nach Anspruch 5, dadurch gekennzeichnet, daß die Flüssigkristall-Mischung (12) mit einem chiralen Wirkstoff dotiert ist.

7. Vorrichtung nach einem der Ansprüche 1 bis 4, dadurch gekennzeichnet, daß das elektro-optische Material ein elektrochromisches Material ist.

8. Vorrichtung nach einem der vorhergehenden Ansprüche, dadurch gekenzeichnet, daß die Scheiben (6, 8) aus einem synthetischen Material hergestellt sind.

9. Vorrichtung nach einem der vorhergehenden Ansprüche, dadurch gekenzeichnet, daß sie zusätzlich Erfassungsmittel (22) umfaßt, die ein für die auf die Erfasungsmittel (22) gelangende Lichtintensität repräsentatives Steuersignal abgeben konnen, wobei die Spannungserzeugungsmittel (18) auf die Transparenz der Zelle (4) in Reaktion auf dieses Steuersignal einwirken.

10. Vorrichtung gemäß Anspruch 9, dadurch gekennzeichnet, daß die opto-elektrische Zelle (4) und die Erfassungsmittel (22) im Verhältnis jeweils zueinander derart angeordnet sind, daß ein Umgebungslicht die Zelle (4) zuerst erreicht.

11. Augenschutzvorrichtung umfassend eine Stützstruktur (2), in welche eine elektro-optische Vorrichtung mit veränderlicher Transparenz (4) montiert ist, dadurch gekennzeichnet, daß die elektro-optische Vorrichtung mit veränderlicher Transparenz (4) gemäß einem der Ansprüche 1 bis 10 ausgebildet ist.

12. Augenschutzvorrichtung nach Anspruch 11, dadurch gekennzeichnet, daß sie zusätzlich eine zweite in die Stützstruktur montierte elektro-optische Zelle aufweist.

13. Augenschutzvorichtung nach Anspruch 11 oder 12, dadurch gekennzeichnet, daß die Spannungserzeugungsmittel durch Solarzellen gebildet sind.
